# EUROPEAN PATENT APPLICATION

(11) **EP 2 441 891 A1**
(43) Date of publication of application: **18.04.2012**
(21) Application number: 10187279.4
(22) Date of filing: 12.10.2010
(51) Int. Cl.: E02B 15/04

(54) **Oil retention booms**

(71) Applicant: Vetco Gray Controls Limited, Bristol BS48 1BS (GB)
(72) Inventor: Packham, Andrew Robert, Chippenham, Wiltshire SN14 0HG (GB)
(74) Representative: Emerson, Peter James

(57) **Abstract**

A boom (1) for retaining oil on the surface (6) of a body of water, is provided with sensing means (12) for detecting such oil and electronic circuitry coupled with the sensing means.

## Description

### Field of the Invention

The present invention relates to oil retention booms.

### Background of the Invention

Oil retention booms are used to try to prevent the spread of leaked or spilled oil on the sea surface by being floated on the sea surface to act a mechanical barrier to oil that has unintentionally leaked or been spilled. Large spills often cover tens if not hundreds of kilometres. Monitoring the dispersal of oil during a spill or leak is a task which can involve large resources, involving spotter aeroplanes and other manual observations and is a very expensive process. This invention endeavours to simplify the task at a much reduced cost.

### Summary of the Invention

According to the present invention from one aspect, there is provided a boom for retaining oil on the surface of a body of water, the boom being provided with sensing means for detecting such oil and electronic circuitry coupled with the sensing means.

According to the present invention from another aspect, there is provided a method of monitoring oil on the surface of a body of water, the method comprising providing an oil retention boom which is provided with sensing means for detecting such oil.

The sensing means could comprise an array of detecting transducers, for example being mounted at an external surface of the boom with the electronic circuitry being carried separately on the boom.

Preferably, the electronic circuitry includes means for processing signals from the transducers, the electronic circuitry preferably being coupled with means for transmitting data resulting from processing said signals. Such transmitting means could comprise wireless transmitting means.

Preferably, there is a plurality of such sensing means spaced apart along the boom.

Preferably, the sensing means and electronic circuitry are adapted for sensing at least one of depth, location and presence of such oil.

The boom could carry a curtain for suspension below the boom, the curtain being provided with a plurality of transducers for detecting oil, which transducers are coupled with the electronic circuitry.

Embodiments of the invention provide an intelligent oil retention boom, in which transducers or devices including transducers are attached to the boom to detect at least one of the presence, location and depth of retained oil and the data from such devices transmitted wirelessly and received at some distance from the boom by a remote receiver, possibly by satellite, to provide detailed information of the location and intensity of oil surface contamination. By way of example, such a receiver could be on a co-ordination vessel for receiving multiple signals from multiple booms.

### Brief Description of the Drawings

Fig. 1a is a plan view of an oil retention boom according to an embodiment of the invention;
Fig. 1b is an enlarged section through A-A of Fig. 1a;
Fig. 2a is a plan view of an oil retention boom according to a second embodiment of the invention; and
Fig. 2b is an enlarged section through A-A of Fig. 2b.

### Description of Embodiments of the Invention

A variety of oil on water detectors are commercially available, ranging from optical devices that detect the chance of reflectivity of the surface of water when oil is floating on it, to capacitive devices that output a change of capacitance when surface oil is present. Ideally sensing means carried by a boom according to the invention will not only detect oil but give an indication of the depth of oil on the surface. Embodiments of the invention propose the use of ultrasonic transducers as detectors for such sensing means, although different detector devices may be utilised, for example ones sensitive to salinity.

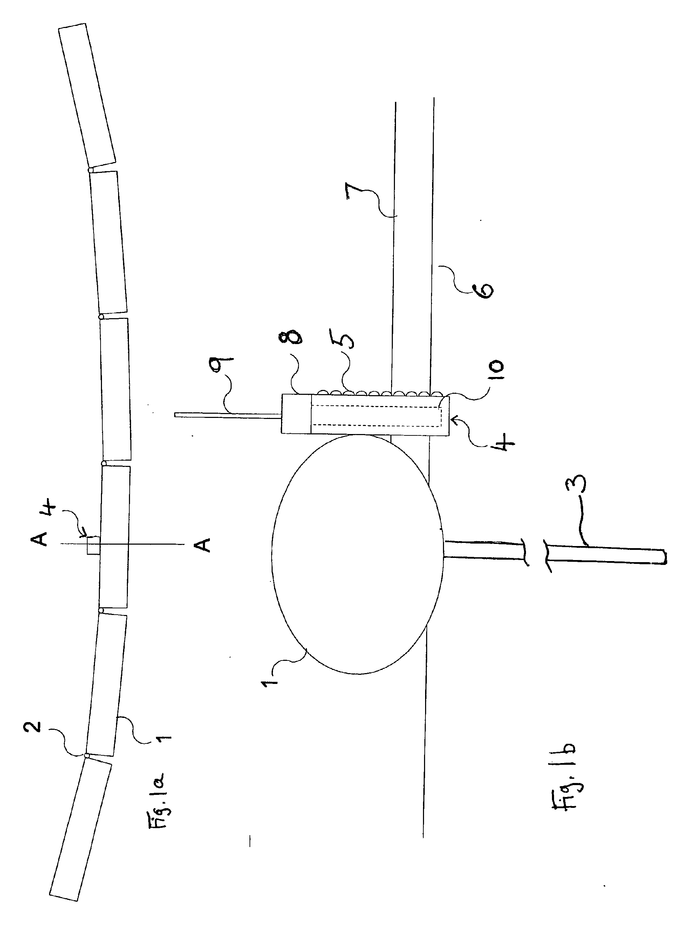

Fig. 1 a is a plan view of a typical oil retaining boom 1 used in the subsea oil production business to retain spilt or leaked oil so that it can be recovered to avoid serious environmental pollution, Fig. 1b being an enlarged section of the boom 1 through A-A of Fig. 1 a. The boom 1 is typically made of sections each filled with buoyancy material, which sections are joined together by hinges 2 and float on the sea surface. A weighted curtain 3 is suspended from the boom 1 into the sea. An intelligent device 4 is attached to the boom 1, which device includes a vertical stack of ultrasonic transducers 5, which are contactable with the sea surface 6 or oil 7 on the sea surface retained by the boom, so that the fluid in contact with a particular transducer acts as a damper on the diaphragm of the transducer. An electrical pulse applied to the transducer induces a natural resonant frequency in the diaphragm, which is dependent on the specific gravity of the particular fluid in contact with diaphragm. Thus, when in contact with water the diaphragm will resonate at a specific frequency (f1) whereas when in contact with oil the diaphragm will resonate at a higher frequency (f2) as the specific gravity of oil is less than that of water. Transducers that are only in contact with air will resonate at a much higher frequency (f3).

Detection of the different frequencies by processing means in electronic circuitry of the device 4, indicates water or oil or air and thus the outputs from the vertically stacked transducers are processed to provide a measure of at least one of the presence of, the location of and the depth of the oil on the sea surface. The integrated output of the stack of transducers 5 is coupled from the processing means to a transmitter 8 of device 4, the transmitter 8 transmitting via an antenna 9 of the device. The device 4 is powered by an internal battery pack 10, which is removable from the device *in situ* and replaced when the batteries are depleted. Typically, the batteries would be of the rechargeable type such as Ni-Mh cells and could be charged by a solar cell mounted on the top of the device 4. The battery pack 10 can be attached to the transmitter 8 and antenna 9 as an assembly with a simple bayonet attachment to the body of the device 4 to facilitate simple replacement in the event of failure (with a simple O-ring seal at the bayonet coupling.) A plurality of such devices 4 could be typically mounted on respective sections of the boom every 10 to 20 metres along the boom.

The transmitters 8 could utilise a mobile phone network if close to land or communicate data via satellite or by conventional UHF radio.

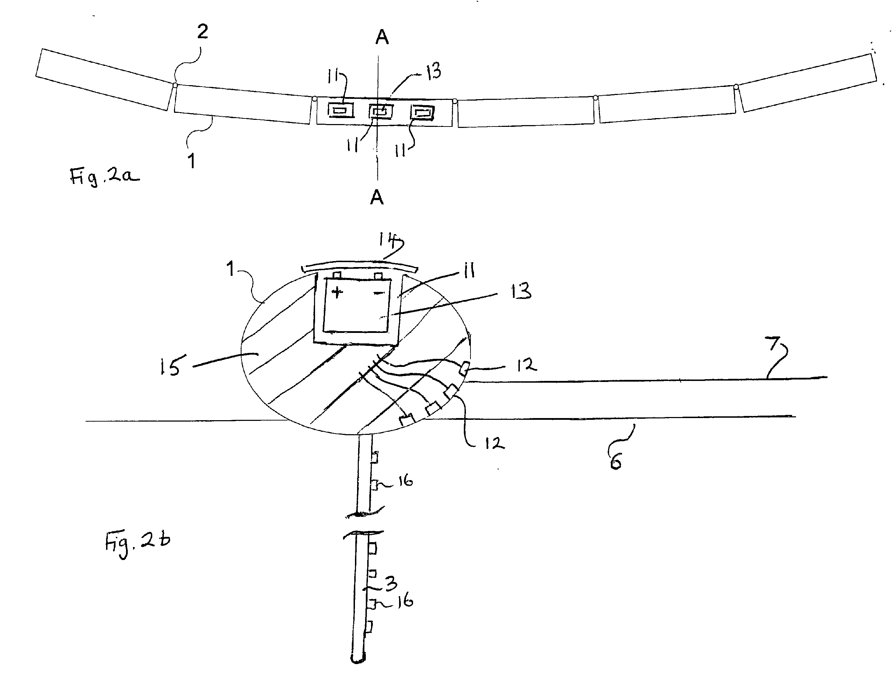

In the above embodiment, the devices 4 are mounted on the boom 1 on its oil retention side. An alternative embodiment will now be described with reference to Figs. 2a and 2b in which items which correspond to those in Figs. 1a and 1b have the same reference numerals as in those figures.

Referring to Figs. 2a and 2b, at least one of the sections of a boom 1 is provided with a plurality of cavities 11, three such cavities being shown by way of example. Each of cavities 11 is for receiving particular equipment. For example, two of them could each receive a respective battery for powering transducers and one of them could receive electronic circuitry including means for processing signals from transducers and a transmitter and antenna for transmitting data. Other arrangements are possible, for example one cavity could receive a battery, another could receive such electronic circuitry and another could receive a transmitter and an antenna. It will be appreciated that there could be fewer than or more than three cavities for receiving one or more batteries, such electronic circuitry, a transmitter and an antenna as desired.

As shown schematically in Fig. 2b, for the or each section of boom 1 having cavities 11, a plurality of transducers 12 are carried at its surface on the oil retention side of the boom. The transducers 12 function in accordance with the transducers 5 of Fig. 1a, being coupled with the electronic circuitry of the section. In Fig. 2b, the particular cavity 11 is shown receiving a battery 13, the cavity having a cover 14. To compensate for the weight of equipment received in cavities 11, the section of boom preferably has an increased physical cross-section compared with sections without cavities 11 and associated equipment, so that there is more buoyancy material (shown by reference numeral 15) to compensate for the weight of the equipment - otherwise the boom would sag where the equipment is. Spaced apart along the curtain 3 are also a plurality of further transducers 16 coupled with the electronic circuitry of the section for use measuring at least one of the presence of, the location of and the depth of deep oil on the sea.

### Advantages of using the Invention

The monitoring of the dispersal of oil during a leak or spill can involve large resources, such as spotter aeroplanes, and other manual observations. This invention enables automation of the task around key areas where the booms are deployed and also enables a more accurate picture of the spread of oil and the highlighting of smaller patches that might be missed.

## Claims

1. A boom for retaining oil on the surface of a body of water, the boom being provided with sensing means for detecting such oil and electronic circuitry coupled with the sensing means.

2. A boom according to claim 1, wherein the sensing means comprises an array of detecting transducers.

3. A boom according to claim 2, wherein the transducers are mounted at an external surface of the boom and the electronic circuitry is carried separately on the boom.

4. A boom according to any preceding claim, wherein the electronic circuitry includes means for processing signals from the transducers.

5. A boom according to claim 4, wherein the electronic circuitry is coupled with means for transmitting data resulting from processing said signals.

6. A boom according to claim 5, wherein said transmitting means comprises wireless transmitting means.

7. A boom according to any preceding claim, wherein there is a plurality of such sensing means spaced apart along the boom.

8. A boom according to any preceding claim, wherein the sensing means and electronic circuitry are adapted for sensing at least one of depth, location and presence of such oil.

9. A boom according to any preceding claim which carries a curtain for suspension below the boom, the curtain being provided with a plurality of transducers for detecting oil, which transducers are coupled with the electronic circuitry.

10. A method of monitoring oil on the surface of a body of water, the method comprising providing an oil retention boom which is provided with sensing means for detecting such oil.

11. A method according to claim 10, wherein the sensing means comprises an array of detecting transducers.

12. A method according to claim 11, wherein the transducers are mounted at an external surface of the boom and the electronic circuitry is carried separately on the boom.

13. A method according to any of claims 10 to 12, wherein the electronic circuitry processes signals from the transducers.

14. A method according to claim 13, wherein the electronic circuitry is coupled with means which transmits data resulting from processing said signals.

15. A method according to claim 14, wherein said means which transmits comprises wireless transmitting means.

16. A method according to any of claims 10 to 15, wherein there is a plurality of such sensing means spaced apart along the boom.

17. A method according to of claims 10 to 16, wherein the sensing means and electronic circuitry sense at least one of depth, location and presence of such oil.

18. A method according to any of claims 10 to 17, wherein the boom carries a curtain suspended below it, the curtain being provided with a plurality of transducers for detecting oil, which transducers are coupled with the electronic circuitry.
